# EUROPEAN PATENT APPLICATION

(11) **EP 0 724 017 A2**
(43) Date of publication of application: **31.07.1996**
(21) Application number: 96101228.3
(22) Date of filing: 29.01.1996
(51) Int. Cl.: C12N 15/56, C12P 13/08, C12P 13/14, C12N 1/19

(54) **Sucrase gene derived from coryneform bacteria**

(30) Priority: 30.01.1995 JP 12361/95
(71) Applicant: Ajinomoto Co., Ltd., Tokyo (JP)
(72) Inventor: Sugimoto, Masakazu, c/o Tech. & Eng. Lab., Kawasaki-shi, Kanagawa-ken (JP); Otsuna, Seiko, c/o Tech. & Eng. Lab., Kawasaki-shi, Kanagawa-ken (JP); Nagase, Kazuo, c/o Cent. Res. Lab., Kawasaki-shi, Kanagawa-ken (JP); Tsuchiya, Makoto, c/o Cent. Res. Lab., Kawasaki-shi, Kanagawa-ken (JP); Hiroshi, Matsui, c/o Cent. Res. Lab., Kawasaki-shi, Kanagawa-ken (JP); Yasuhiko, Yoshihara, c/o Tech. & Eng. Lab., Kawasaki-shi, Kanagawa-ken (JP); Nakamatsu, Tsuyoshi, c/o Tech. & Eng. Lab., Kawasaki-shi, Kanagawa-ken (JP)
(74) Representative: Strehl Schübel-Hopf Groening & Partner

(57) **Abstract**

The present invention provides a DNA fragment derived from Coryneform bacteria and containing a gene coding for a protein having sucrase activity and a recombinant DNA containing said DNA fragment and capable of gene amplification in Coryneform bacteria. The recombinant DNA is introduced into Coryneform bacteria to enhance their sucrase activity. By using the bacteria having enhanced sucrase activity a method is provided for efficiently producing L-amino acids and nucleic acids in a short period of time.

## Description

### Field of the Invention:

The present invention relates to a sucrase gene derived from Coryneform bacteria. More particularly, the present invention relates to a DNA fragment derived from Coryneform bacteria and containing a gene coding for a protein having sucrase activity, a recombinant DNA obtained by inserting said DNA fragment into a vector capable of gene amplification in Coryneform bacteria, a microorganism belonging to Coryneform bacteria harboring said recombinant DNA and capable of producing an L-amino acid or a nucleic acid, and a method for production of an L-amino acid or a nucleic acid by culturing said microorganism.

### Discussion of the Background:

Many studies have heretofore been reported regarding L-amino acid-producing microorganisms prepared using gene engineering technology. See Biotechnology Letters, Vol. 2, pp. 525-530 (1980); Appl. Environ. Microbiol., Vol. 144, pp. 181-190 (1979); and Preprint of Discussions and Lectures in the Agricultural and Chemical Society of Japan (1981), pp. 8, all incorporated herein by reference. These studies all concern the amplification of genes for the biosynthesis of L-amino acids, thereby increasing per cell productivity for the intended L-amino acids. The amplification does not increase the efficiency of L-amino acid production by enhancing the assimilation of saccharides, the raw materials to be fermented, by the microorganism.

There are some examples referring to the enhancement of the assimilation of saccharides by L-amino acid-producing microorganisms, using gene engineering technology. See Japanese Patent Application Laid-open Nos. 61-119185 and 2-171178 incorporated herein by reference. According to these studies, *Escherichia coli* was provided with the ability to assimilate sucrose by gene manipulation. In particular, a strain of *Escherichia coli* was provided with sucrose uptake activity, later hydrolyzing the sucrose into glucose and fructose, with the result that the strain was capable of utilizing sucrose as a carbon source, an ability which the strain did not possess inherently. No one has reported similar success with Coryneform bacteria, however, and no publication regarding the enhanced ability of Coryneform bacteria to assimilate saccharides such as sucrose, etc., using gene engineering technology has yet appeared.

### OBJECTS OF THE INVENTION

One object of the present invention is to improve the sucrose assimilating ability of bacteria by means of gene engineering technology, to thereby increase the speed of fermentation with the altered bacteria and so as to improve the productivity of L-amino acids and nucleic acids from raw materials containing sucrose. More particularly, the objects of the present invention include obtaining a DNA fragment derived from Coryneform bacteria and containing a gene coding for a protein having sucrase activity, a recombinant DNA comprising said DNA fragment, a vector capable of gene amplification in Coryneform bacteria comprising said fragment, a microorganism, particularly a Coryneform bacteria, harboring said recombinant DNA, a microorganism, particularly a Coryneform bacteria, harboring said vector, and a method for improving the efficiency of L-amino acid or a nucleic acid production by fermentation of transformed microorganism, particularly transformed Coryneform bacteria, in the presence of raw materials containing sucrose.

### DETAILED DESCRIPTION OF THE PREFERRED EMBODIMENT

The present inventors have succeeded in obtaining the above objects and more by discovering DNA fragments derived from Coryneform bacteria and containing a gene coding for a protein having sucrase activity. In addition, they have discovered a method for producing L-amino acids and nucleic acids in high yield and/or in a shorter period of time than conventional methods by culturing Coryneform bacteria into which recombinant DNA containing the DNA fragment described above had been introduced.

Specifically, the present invention provides a DNA fragment derived from Coryneform bacteria and containing a gene coding for a protein having sucrase activity, a vector capable of gene amplification in Coryneform bacteria and comprising said DNA fragment, a microorganism belonging to Coryneform bacteria harboring said vector and capable of producing an L-amino acid or a nucleic acid, a recombinant DNA obtained by inserting said DNA fragment into a vector capable of gene amplification in Coryneform bacteria and a method for the production of an L-amino acid or a nucleic acid comprising culturing said microorganism.

The term "protein having sucrase activity" as used herein refers to a protein capable of hydrolyzing sucrose to produce glucose and fructose. The protein preferably has a sucrase activity of at least 1 unit/mg of protein, preferably an activity of 2, 3, 4, or 5 or more units/mg of protein.

The term "Coryneform bacteria" as referred to herein indicates aerobic gram-positive rods belonging to the genus *Microbacterium*, *Brevibacterium* or *Corynebacterium* as described in Bergey's manual of Determinative Bacteriology, 8th Ed., p. 599 (1974), incorporated herein by reference, and includes the following wild strains capable of producing L-glutamic acid and mutant strains derived therefrom:
- *Corynebacterium acetoacidophilum*: ATCC 13870
- *Corynebacterium acetoglutamicum*: ATCC 15806
- *Corynebacterium callunae*: ATCC 15991
- *Corynebacterium glutamicum*: ATCC 13032
- *Corynebacterium glutamicum*: ATCC 13060
- *Brevibacterium divaricatum*: ATCC 14020
- *Brevibacterium lactofermentun*: ATCC 13869
- *Corynebacterium lilium*: ATCC 15990
- *Corynebacterium melassecola*: ATCC 17965
- *Brevibacterium saccharolyticum*: ATCC 14066
- *Brevibacterium immariophilum*: ATCC 14068
- *Brevibacterium roseum*: ATCC 13825
- *Brevibacterium flavum*: ATCC 13826
- *Brevibacterium thiogenitalis*: ATCC 19240
- *Microbacterium ammoniaphilum*: ATCC 15354
- *Brevibacterium ammnoniagenes*: ATCC 6872
- *Corynebacterium equi*: ATCC 21280

The gene coding for a protein having sucrase activity as referred to herein indicates a gene which codes for a protein having the above described activity for hydrolyzing sucrose to produce glucose and fructose. Sucrase activity is important for microorganisms when they utilize sucrose as a carbon source. The gene is not otherwise limited. Certain preferred base sequences identified herein do not define the scope of this invention. The preferred base sequences, their functional equivalents, particularly those having up to ± 300 additional or fewer bases, 3' or 5' or both, as compared to the preferred sequences as well as the ability to code for a protein having at least 75% of the sucrase activity that the protein coded for by the preferred sequence exhibits, and any other gene meeting the above functional requirements are included herein. Therefore, the present invention also relates to proteins having amino acid substitutions and/or insertions and/or deletions and having at least 75 % of the sucrase activity of the protein encoded by the sequence from bases 2,338 to 3,609 of base sequence ID No. 4.

To obtain a DNA fragment containing a gene coding for a protein having sucrase activity according to the present invention, a method comprising construction of a gene library from chromosomal DNA of Coryneform bacteria may be used followed by the cloning of a sucrase gene by hybridization. This will be described in detail hereunder.

A gene library of the Coryneform genome can be constructed using conventional techniques such as those discussed by Sambrook et al, Molecular Cloning: A laboratory manual, 2nd ed., CSH press, NY, 1989 incorporated herein by reference, particularly chapters 1 and 9. Briefly, chromosomal DNA obtained from Coryneform bacteria is partially digested with a restriction endonuclease, ligated into a suitable cloning vector such as a plasmid and transformed into a suitable host. A preferred cloning vector for construction of the Coryneform genomic library is pUC18. A preferred host is E.coli, particularly E.coli JM1O9.

Sucrase or invertase genes have been cloned with *Bacillus subtilis* (Gene, Vol. 45, pp. 221-225 (1986) incorporated herein by reference), *Zymomonas mobilis* (J. Bacteriol., Vol. 172, pp. 6727-6735 (1990) incorporated herein by reference), *Streptococcus mutans* (Infect. Immun., Vol. 56, pp. 1956-1960 (1988) incorporated herein by reference), yeasts of the genus *Saccharomyces* (Nucleic Acids Res., Vol. 11, pp. 1943-1954 (1983) incorporated herein by reference), *Vibrio alginolyticus* (Gene, Vol. 80, 49-56 (1989) incorporated herein by reference), etc., and these genes are homologous to one another in some degree (J. Bacteriol., Vol. 172, pp. 6727-6735 (1990) incorporated herein by reference). Synthetic DNAs can be prepared, using conventional means such as solution or solid phase DNA synthesis preferably using an automated DNA synthesizer, on the basis at the portions common to these microorganisms.

These synthetic DNAs thus formed are phosphorylated, for example by using [γ-³²P]ATP as the substrate, to prepare labeled probes. The library formed above is then screened via colony hybridization using the probes. From the colonies hybridizing with the probes, those expressing sucrase activity are selected. The active DNA fragment is then excised from the cloning vector by conventional techniques.

The expression vector comprising the DNA fragment containing a gene coding for a protein having sucrase activity, which is employed in the present invention, may be any suitable plasmid replicable in microorganisms, e.g. Coryneform bacteria. For example useful vectors include pHM1519 (Agric., Biol. Chem., Vol. 48 (1984), pp. 2901-2903 incorporated herein by reference), pAM330 (Agric. Biol- Chem., Vol. 48 (1984), pp. 2901-2903 incorporated herein by reference), drug-resistant plasmids based on these, etc. The vector may be introduced in chromosomes of microorganisms by homologous recombination or through insertion sequences. As to homologous recombination are known chromosome gene recombination method in Corynebacteria using temperature sensitive replication origin; see Japanese Patent Application Laid-Open No. 5-7491. As to insertion sequences are known IS714, IS719, IS903 and so on, and these sequences are used for the above purpose; see WO93/18151.

Preferred host bacteria are Coryneform bacteria. Examples of such host bacteria to be used in the present invention include wild strains of L-glutamic acid-producing bacteria and mutant strains derived therefrom and capable of producing another L-amino acid or a nucleic acids such as inosine, 5'-inosinic acid, guanosine and 5'-guanylic acid. The L-amino acids include those produced by Coryneform bacteria, including, for example, L-glutamic acid, L-lysine, L-threonine, L-aspartic acid, L-isoleucine, L-arginine, L-proline, L-histidine, L-valine, L-leucine, L-phenylalanine, L-glutamine, etc.

Specific examples of hosts which are suitable for producing various L-amino acids and nucleic acids are mentioned below.
(i) Hosts preferred for producing L-glutamic acid:,
   *Brevibacterium lactofermentum* AJ 12745 (FERM BP-2922); see U.S. Patent No. 5,272,067, page 561.
   *Brevibacterium lactofermentum* AJ 12746 (FERM BP-2923): see U.S. Patent No. 5,272,067.
   *Brevibacterium lactofermentum* AJ 12747 (FERM BP-2924); see U.S. Patent No. 5,272,067.
   *Brevibacterium lactofermentum* AJ 12748 (FERM BP-2925); see U.S. Patent No. 5,272,067.
   *Corynebacterium glutamicum* ATCC 21942; see Japanese Patent Application Laid-Open No. 5-3793, page 3, Table 1.
   In addition to these and others, wild strains of Coryneform bacteria mentioned below can also be employed as a host.
   - *Corynebacterium acetoacidophilum*: ATCC 13870
   - *Corynebacterium acetoglutamicum*: ATCC 15806
   - *Corynebacterium callunae*: ATCC 15991
   - *Corynebacterium glutamicum*: ATCC 13032
   - *Corynebacterium glutamicum*: ATCC 13060
   - *Brevibacterium divaricatum*: ATCC 14020
   - *Brevibacterium lactofermentum*: ATCC 13869
   - *Corynebacterium lilium*: ATCC 15990
   - *Corynebacterium melassecola*: ATCC 17965
   - *Brevibacterium saccharolyticum*: ATCC 14066
   - *Brevibacterium immariophilum*: ATCC 14068
   - *Brevibacterium roseum*: ATCC 13825
   - *Brevibacterium flavum*: ATCC 13826
   - *Brevibacterium thiogenitalis*: ATCC 19240
   - *Microbacterium ammoniaphilum*: ATCC 15354
(ii) Hosts preferred for producing L-lysine:
   *Brevibacterium lactofermentum* AJ 12031 (FERM BP-277); see Japanese Patent Application Laid-Open No. 60-62994, page 525, left bottom column.
   *Brevibacterium lactofermentum* ATCC 39134; see Japanese Patent Application Laid-Open No. 60-62994, page 473, right bottom column.
   *Brevibacterium lactofermentum* AJ 11082 (NRRL B-11470, FERM P-3840); see U.S. Patent No. 4,275,157
   *Brevibacterium lactofermentum* AJ 12435 (FERM BP-2294); see U.S. Patent No. 5,304,476
   *Brevibacterium lactofermentum* AJ 12592 (FERM BP-3239); see U.S. Patent No. 5,304,476
   *Brevibacterium lactofermentum* AJ 12593 (FERM BP-3240); see U.S. Patent No. 5,304,476
   *Corynebacterium glutamicum* AJ 12596 (FERM BP-3242); see U.S. Patent No. 5,304,476
   *Corynebacterium glutamicum* AJ 3463 (FERM P-1987); see Japanese Patent Publication No. 51-34477.
(iii) Hosts preferred for producing L-threonine:
   *Brevibacterium lactofermentum* AJ 11188 (FERM P-4190); see Japanese Patent Application Laid-Open No. 60-87788, page 473, right top column.
   *Corynebacterium glutamicum* AJ 11682 (FERM BP-118); see Japanese Patent Publication No. 2-31956, page 230, column 8.
   *Brevibacterium flavum* AJ 11683 (FERM BP-119); see Japanese Patent Publication No. 2-31956, page 231, column 10.
(iv) Hosts preferred for producing L-aspartic acid:
   *Brevibacterium flavum* AJ 3859 (FERM P-2799); see Japanese Patent Application Laid-Open No. 51-61689, page 524, left top column.
   *Brevibacterium lactofermentum* AJ 3860 (FERM P-2800); see Japanese Patent Application Laid-Open No. 51-61689, page 524, left top column.
   *Corynebacterium acetoacidophilum* AJ 3877 (FERM P-2803); see Japanese Patent Application Laid-Open No. 51-61689, page 524, left top column.
   *Corynebacterium glutamicum* AJ 3876 (FERM P-2802); see Japanese Patent Application Laid-Open No. 51-61689, page 524, left top column.
(v) Hoses preferred for producing L-isoleucine:
   *Brevibacterium lactofermentum* AJ 12404 (FERM P-10141) (see Japanese Patent Application Laid-Open No. 2-42988, page 603, left bottom column).
   *Brevibacterium flavum* AJ 13405 (FERM P-10142) (see Japanese Patent Application Laid-Open No. 2-42988, page 524, left bottom column).
(vi) Hosts preferred for producing L-arginine:
   *Brevibacterium flavum* AJ 12144 (FERM P-7642); see Japanese Patent Publication No. 5-27388, page 174, column 4.
   *Corynebacterium glutamicum* AJ 12145 (FERM P-7643); see Japanese Patent Publication No. 5-27388, page 174, column 4.
   *Brevibacterium flavum* ATCC 21493; see Japanese Patent Application Laid-Open No. 5-3793, page 3, Table 1.
   *Corynebacterium glutamicum* ATCC 21659; see Japanese Patent Application Laid-Open No. 5-3793, page 3, Table 1.
(vii) Hosts preferred for producing L-proline:
   *Brevibacterium lactofermentum* AJ 11225 (FERM P-4370); see Japanese Patent Application Laid-Open No. 60-87788, page 473, left top column.
   *Brevibacterium flavum* AJ 11512 (FERM P-5332); see Japanese Patent Publication No. 62-36679, page 185, column 2.
   *Brevibacterium flavum* AJ 11513 (FERM P-5333); see Japanese Patent Publication No. 62-36679, page 185, column 2.
   *Brevibacterium flavum* AJ 11514 (FERM P-5334); see Japanese Patent Publication No. 62-36679, page 185, column 2.
   *Corynebacterium glutamicum* AJ 11522 (FERM P-5342); see Japanese Patent Publication No. 62-36679, page 185, column 2.
   *Corynebacterium glutamicum* AJ 11523 (FERM P-5343); see Japanese Patent Publication No. 62-36679, page 185, column 2.
(viii) Hosts preferred for producing L-histidine:
   *Brevibacterium flavum* AJ 3420 (FERM P-2316); see U.S. Patent No. 5,294,547
   *Brevibacterium flavum* AJ 12425 (FERM BP-2212); see U.S. Patent No. 5,294,547
   *Corynebacterium glutamicum* AJ 12092 (FERM P-7273); see U.S. Patent No. 5,294,547
   *Corynebacterium glutamicum* AJ 12426 (FERM BP-2213); see U.S. Patent No. 5,294,547
(ix) Hosts preferred for producing L-valine:
   *Brevibacterium lactofermentum* AJ 3434 (FERM P-1845); see U.S. Patent No. 5,188,948
   *Brevibacterium lactofermentum* AJ 12341 (FERM BP-1763); see U.S. Patent No. 5,188,948
   *Corynebacterium glutamicum* AJ 3776 (FERM P-2601); see U.S. Patent No. 5,188,948
   *Corynebacterium glutamicum* AJ 12342 (FERM BP-1764); see U.S. Patent No. 5,188,948
(x) Hosts preferred for producing L-leucine:
   *Brevibacterium lactofermentum* AJ 3452 (FERM P-1965); see U.S. Patent No. 3,970,519
   *Brevibacterium lactofermentum* AJ 3719 (FERM P-2517); see U.S. Patent No. 3,970,519
   *Corynebacterium glutamicum* AJ 3453 (FERM P-1966); see U.S. Patent No. 3,970,519
   *Corynebacterium glutamicum* AJ 3455 (FERM P-1968); see U.S. Patent No. 3,970,519
(xi) Hosts preferred for producing L-phenylalanine:
   *Brevibacterium lactofermentum* AJ 12637 (FERM BP-4160); see Japanese Patent Application Laid-Open No. 5-49489
   *Corynebacterium acetoacidophilum* AJ 11761 (FERM P-6286); see Japanese Patent Publication No. 2-11235
   *Corynebacterium acetoacidophilum* AJ 12638 (FERM P-12382); see Japanese Patent Publication No. 2-11235
(xii) Hosts preferred for producing L-glutamine:
   *Brevibacterium flavum* AJ 12418 (FERM BP-2205); see U.S. Patent No. 5,294,547
   *Corynebacterium acetoacidophilum* AJ 12419 (FERM BP-2206); see U.S. Patent No. 5,294,547
(xiii) Hosts preferred for producing 5'-inosinic acid:
   *Brevibacterium ammoniagenes* AJ 12192 (FERM P-7949); see Japanese Patent Publication No. 5-998
   *Corynebacterium equi* AJ 11347 (FERM P-4968); see Japanese Patent Publication No. 57-22558
   *Corynebacterium equi* AJ 11350 (FERM P-4971); see Japanese Patent Publication No. 57-22558
   *Corynebacterium equi* AJ 11352 (FERM P-4973); see Japanese Patent Publication No. 57-22558

All of the above Japanese Patent Publications, Japanese Applications and U.S. Patents are incorporated herein by reference.

To introduce the vector or recombinant DNA comprising a gene coding for a protein having sucrase activity into the above-mentioned Coryneform bacteria, well-known methods such as the protoplast method (Gene, Vol. 39, pp.281-286 (1985) incorporated herein by reference), an electroporation method (Bio/Technology, Vol. 7, pp. (1989); Japanese Patent Application Laid-Open No. 2-207791 incorporated herein by reference), etc. can be used.

Accordingly, any suitable vector or recombinant DNA comprising a gene coding for a protein having sucrase activity may be used to transform other microorganisms and to express sucrase activity therein.

The method for culturing the above-mentioned hosts harboring the vector or recombinant DNA need not be different from conventional methods for culturing general L-amino acid-producing microorganisms. For instance, the media to be used for the cultivation may be any conventional one containing carbon sources, nitrogen sources, inorganic ions and, if desired, organic minor nutrients such as amino acids, vitamins, etc.

Typical carbon sources include glucose, sucrose, etc., as well as liquid hydrolysates of starch, molasses, etc., containing them. However, the advantage of the present invention is made clear when cultivation is performed in a medium containing sucrose as the carbon source. That is, the production speed of L-amino acids and nucleic acids is remarkably increased as compared with prior methods when raw materials containing sucrose such as cane molasses and beet molasses are fermented. As the nitrogen sources, ammonia gas, aqueous ammonia, ammonium salts, etc. may be used. When nutritional mutant strains which require amino acids, etc. are used as the hosts, it is necessary to add nutrients such as amino acids, etc. which are required by the strains to the media to be used for culturing them.

The cultivation is preferably performed aerobically, while controlling the pH of the culture between 6.0 and 8.0 and the fermentation temperature between 25°C and 40°C, until the production and the accumulation of the intended L-amino acid or nucleic acid are substantially stopped. To collect the L-amino acid or nucleic acid thus accumulated in the culture, any ordinary method may be used such as crystallization, ion-exchange methods, etc.

The present invention will now be further described by reference to the following examples. The invention is not limited thereto, however.

### Example 1

### (Construction of Gene Library)

Chromosomal DNA was prepared from *Brevibacterium lactofermentum* ATCC 13869, according to a Saito-Miura method (Biochem. Biophs. Acte., Vol. 8278, pp. 619-629 (1963) incorporated herein by reference). About 100 µg of the thus-prepared DNA was partially digested with Sau3Al and charged into a sucrose density gradient of from 10% to 40% and subjected to ultra-centrifugation at 22,000 rpm for 22 hours at 20°C. The bottom of the tube was punctured with a needle, through which one ml aliquots were fractionated to obtain a fraction having approximately from 1500 to 6000 bp (base pairs). This fraction was dialyzed against TE buffer (10 mM Tris, 1 mM EDTA, pH 8.0) and ligated with vector pUC18 that had been cut with BamHl, using T4DNA ligase. *Escherichia coli* JM 109 was transformed by the resulting DNA. As a result, a gene library comprising about 10,000 clones was constructed.

### (Designation of Probes)

The base sequences of the genes of sucrase, levanase and invertase mentioned below have already been determined. These include levanase (Mol. Genet., Vol. 208, pp. 177-184 (1987) incorporated herein by reference) and sucrase (Gene, Vol. 45, pp. 221-225 (1986) incorporated herein by reference), all derived from *Bacillus subtilis*; sucrase derived from *Streptococcus mutans* (Infect. Immun., Vol. 56, pp. 1956-1960 (1988) incorporated herein by reference); invertase derived from *Saccharomyces cerevisias* (Nucleic Acids Res., Vol. 11, pp. 1943-1954 (1983) incorporated herein by reference); sucrase derived from *Zymomonas mobilis* (J. Bacteriol., Vol. 172 (1990), pp. 6727-6735 incorporated herein by reference); and sucrase derived from V*ibrio alginolyticus* (Gene, Vol. 80, pp. 49-56 (1989), incorporated herein by reference). The sequences of these genes are partly homologous to one another (J. Bacteriol., Vol. 172, pp. 6727-6735 (1990) incorporated herein by reference). Based on this information, DNAs each having the base sequence selected from SEQ ID NOs: 1, 2 and 3 were designed and synthesized, using a DNA synthesizer, to be used as probes.

### (Hybridization)

The library formed above was used for colony hybridization to screen for colonies hybridizing with the probes, according to a Derek method (FOCUS, Vol. 6, pp. 1-4 (1984) incorporated herein by reference). The temperature for the hybridization was 50°C and that for washing the filters was 48°C. The clone hybridizing with each probe was cultured in 100 ml of an M9 medium (Molecular Cloning, 2nd Ed., Cold Spring Harbor Press (1989); Maniatis, et al-, A.3 in Appexndixes incorporated herein by reference). The collected cells were washed with 0.9 % physiological saline solution, then suspended in 5 ml of 0.1 M sodium phosphate buffer (pH 7.3) and thereafter disrupted by ultrasonication. The suspension was then centrifuged at 33,000 rpm for one hour to separate the resulting supernatant and provide a crude extract. 150 µl of the crude extract were mixed with 50 µl of 0.5 M sucrose and reacted at 30°C for one hour, and then heated at 100°C for 3 minutes to inactivate the enzyme. The glucose produced was detected, using the Glucose C Test Wako (made by Wako Pure Chemicals Co.), to determine the sucrase activity. The strain of one clone detected with the probe having the sequence of SEQ ID NO:2 showed a strong sucrase activity (Table 1). Table 1 shows the sucrase activity (as relative activity) per mg of the protein. One unit is defined as the activity necessary to produce 1 mg of glucose in one hour at 30°C. The plasmid in the strain providing the sucrase activity is referred to as pBS3-43.

**Table 1**

| Plasmid/Host | Sucrase Activity (unit/mg protein) |
|---|---|
| pUC18/JM109 | 0 |
| pBS3-43/JM109 | 3.1 |

### (Analysis of Clone)

The SmaI fragment of about 6 kb was cleaved out of the inserted DNA and this was inserted into the SmaI site of pSAC4, a shuttle vector for *Escherichia coli* and Coryneform bacteria which imparts resistance to chloramphenicol to the host. The resulting plasmid was named as pSSM30 and expressed sucrase activity in *Escherichia coli*(data not shown).

*Escherichia coli* JM109 having plasmid pSSM30 was named as AJ13047 and deposited under the Budapest Treaty in the National Institute of Bioscience and Human-Technology, Agency of Industrial Science and Technology, 1-3, Higashi 1 chome, Tsukuba-shi, Ibarakiken 305, Japan, to which the deposition number of FERM BP-4800 was rendered.

*Brevibacterium lactofermentum* ATCC13869 was transformed with pSSM30 using electro puls method (Japanese Patent Laid-Open No. 2-207791). The transformant having pSSM30 was named as ATCC13869/pSSM30. ATCC13869/pSSM30 was cultured in 100 ml of a CM2G medium (yeast extract 10g/l, polypeptone 10g/l, glucose 5g/l, NaCl 5g/l, pH 7.0). The collected cells were washed with 0.9 % physiological saline solution, then suspended in 5 ml of 0.1 M sodium phosphate buffer (pH 7.3) and thereafter disrupted by ultrasonication. The suspension was then centrifuged at 100,000xG to separate the resulting supernatant and provide a crude extract. 150 µl of the supernatant was mixed with 50 µl of 0.5 M sucrose and reacted at 30°C for one hour, and then heated at 100°C for 3 minutes to inactivate the enzyme. The glucose produced was detected, using the Glucose C Test Wako (made by Wako Pure Chemicals Co.), to determine the sucrase activity. As *Brevibacterium lactofermentum* ATCC13869 originally has sucrase activity in its cell body, transformant in which *Brevibacterium lactofermentum* ATCC13869 is the host and pSAC4 is the plasmid was used as control. The result is shown in Table 2. Table 2 shows the sucrase activity (as relative activity) per mg of the protein. One unit is defined as the activity necessary to produce 1 mg of glucose in one hour at 30°C. As shown in Table 2, the strain having pSSM30 was strengthened in its sucrase activity.

**Table 2**

| Host/Plasmid | Sucrase Activity (unit/mg protein) |
|---|---|
| ATCC13869/pSSM30 | 5.7 |
| ATCC13869/pSAC4 | 0.72 |

### (Determination of Base Sequence)

About 7 kb of the DNA fragments including the above-mentioned SmaI fragment which was inserted into pSSM30 were sequenced by a fluorescent labeling method to determine their base sequence. The sequence is represented by SEQ ID NO:4. There were four open reading frames in the sequence (ORF-Fl: from 342nd to 1,505th., ORF-F2: from 2,338th to 3,609th; ORFF3: from 4,438th to 5,358th; ORF-F4: from 5,570th to 6,577th). By the homology between the gene and a known sucrase gene, the structural gene of sucrase was presumed to be ORF-F2, coding for 424 amino acid residues. The other presumed open reading frames were examined to detect the homology, if any, by protein data base NBRF, and the results shown in Table 3 were obtained. ORF-F3 and ORF-F4 were found to have high homology therebetween, and there will be some relation between the two.

**Table 3**

| ORF No. | Number of Amino Acids | Name (homologous protein of different kind) | Degree of Homology (%) |
|---|---|---|---|
| F1 | 388 | N-acetylglucosamine-6-phosphate deacetylase *E. coli* (382a.a) | 24 |
| F3 | 307 | UDP-N-acetylmuramoylalany l-D-glutamyl-meso-6-diaminopimelate synthetase B.sub (494a.a) | 36 |
| F4 | 336 | Phospho-N-acetylmuramoylpentapeptidetransferase B.sub (324a.a) | 39 |

### Example 2

(Introduction of sucrase gene plasmid into L-lysine-producing bacteria) Sucrase gene plasmid pSSM30 was prepared from *Escherichia coli* AJ13047 (FERM BP-4800) and introduced into L-lysine-producing bacteria of *Brevibacterium lactofermentum* AJ 11082 (NRRL B-11470), using an electric pulse method (see Japanese Patent Application Laid-Open No. 2-207791 incorporated herein by reference). The cells were grown on an M-CM2G plate (containing 5 g of glucose, 10 g of polypeptone, 10 g of yeast extract, 5 g of NaCl, 0. 2 g of DL-methionine and 15 g of agar in one liter of distilled water and having pH of 7.2) containing 5 µg/ml of chloramphenicol, thereby selecting the intended transformant therefrom. The thus-obtained transformant was referred to as AJ 11082/pSSM30.

### (Production of L-lysine from sucrose, using sucrase gene-amplified bacteria)

AJ 11082 and AJ 11082/pSSM30 were separately cultured in a medium containing sucrose to produce L-lysine by fermentation. The cells were inoculated in a one-liter jar fermenter containing 300 ml of a medium for lysine production containing sucrose as the carbon source (i.e., containing 100 g of sucrose, 55 g of (NH₄)₂SO₄, 1 g of KH₂PO₄, 1 g of MgSO₄.7H₂0, 50 ml of hydrolysate of soybean protein, 10 mg of FeSO₄.7H₂O, 10 mg of MnSO₄.4H₂O and 5 mg of nicotinic acid amide in one liter of distilled water) and the cultivation was performed at 31.5°C and at pH 7.0 (adjusted with ammonia gas) with agitation and aeration. After the sucrose originally added to the medium was wholly consumed by the cells, a mixture comprising 60 g/dl of sucrose and 8 g/dl of ammonium sulfate, that had been sterilized separately, was continuously fed into the fermenter so that the sucrose concentration in the culture was controlled to fall within the range of from 1 g/dl to 3 g/dl. The cultivation was continued until 150 ml of the feed solution was consumed. Table 4 shows the cultivation time and the amount of L-lysine accumulated. Due to the amplification of the sucrase gene, the speed at which L-lysine was produced by fermentation of sucrose was significantly increased. After the cultivation, a crude enzyme was extracted from the cells by a known method and its sucrase activity was measured.
This result is also shown in Table 4.

**Table 4**

| Strain | Cultivation Time (hour) | Amount of L-lysine Accumulated (g/dl) | Sucrase Activity (unit/mg protein) |
|---|---|---|---|
| AJ11082 | 65 | 9.5 | 0.35 |
| AJ11082/pSSM30 | 34 | 9.4 | 3.54 |

### (Example 3)

### (Introduction of sucrase gene plasmid into wild strain of Brevibacterium lactofermentum)

Sucrase gene plasmid pSSM30 was introduced into a wild strain of *Brevibacterium lactofermentum* ATCC 13869, using an electric pulse method (see Japanese Patent Application Laid-Open No. 2-207791 incorporated herein by reference). The cells were grown on an M-CM2G plate (containing 5 g of glucose, 10 g of polypeptone, 10 g of yeast extract, 5 g of NaCl, 0.2 g of DL-methionine and 15 g of agar in one liter of distilled water and having pH of 7.2) containing 5 µg/ml of chloramphenicol, thereby selecting the intended transformant therefrom. The thus-obtained transformant was referred to as ATCC 13869/pSSM30.

### (Production of L-glutamic acid from sucrose, using sucrase gene-amplified bacteria)

ATCC 13869 and ATCC 13869/pSSM30 were separately cultured in medium containing sucrose to produce L-glutamic acid by fermentation. The cells were inoculated in one-liter jar fermenters containing 300 ml of a medium for the L-glutamic acid production containing sucrose as the carbon source (i.e., containing 100 g of sucrose, 15 g of (NH₄)₂SO₄, 2.5 g of KH₂PO₄, 0.4 g of MgSO₄.7H₂0, 50 ml of hydrolysate of soybean protein, 10 mg of FeSO₄.7H₂O, 10 mg of MnSO₄.4H₂0, 350 µg of thiamine.HCl and 3.5 µg of biotin in one liter of distilled water) and cultured at 31.5°C and at pH 6.5 (adjusted with ammonia gas) with agitation and aeration. After the sucrose originally added to the medium was wholly consumed by the cells, a mixture comprising 60 g/dl of sucrose and 8 g/dl of ammonium sulfate, that had been sterilized separately, was continuously fed into the fermenter such that the sucrose concentration in the culture was controlled to fall within the range of from 1 g/dl to 3 g/dl. Thus, the cultivation was continued until 150 ml of the feed solution was consumed. Table 5 shows the cultivation time and the amount of L-glutamic acid accumulated. Due to the amplification of the sucrase gene, the speed at which L-glutamic acid was produced by fermentation of sucrose was significantly increased.

**Table 5**

| Strain | Cultivation Time (hour) | Amount of L-glutamic Acid Accumulated (g/dl) |
|---|---|---|
| ATCC13869 | 45 | 10.7 |
| ATCC13869/pSSM30 | 29 | 10.9 |

### Example 4

### (Reduction of sucrase gene fragment)

To identify the region essential for increasing the sucrose-metabolizing speed in pSSM30, the DNA fragment was reduced. A gene fragment of about 1.5 kb containing from SacII site starting from the 2,155th base to BanI site starting from the 3,722nd base in the base sequence of SEQ ID NO:4, which contains ORF-F2 presumed to code for the sucrase activity, was extracted from pSSM30, and its both ends were made blunt using T4 DNA polymerase. This fragment was introduced into the SmaI site of pHSG399, a vector for *E. coli*. In addition, so as to make the plasmid replicable in the cells of Coryneform bacteria, the replication origin of a plasmid of *Corynebacterium glutamicum* was introduced thereinto. Specifically, from plasmid pHC4 (see Japanese Patent Application Laid-Open No. 5-7491 incorporated herein by reference) of *Corynebacterium glutamicum* having a replication origin therein, a DNA fragment of 3 kb containing the replication origin was extracted, and its both ends were made blunt using T4 DNA polymerase, followed by modifying the restriction enzyme sites into BamHI site with linkers. This fragment was inserted into the BamHI site of pHSG399 ligated with the sucrase gene of about 1.5 kb. The thus-constructed plasmid was referred to as pSSM30BS.

### Example 5

### (Introduction of reduced sucrase gene plasmid into L-lysine-producing bacteria)

The reduced sucrase gene plasmid pSSM30BS was introduced into an L-lysine-producing strain of *Brevibacterium lactofermentum* AJ 11082 (NRRL B-11470), using an electric pulse method (see Japanese Patent Application Laid-Open No. 2-207791). The cells were grown on an M-CM2G plate (containing 5 g of glucose, 10 g of polypeptone, 10 Cl of yeast extract, 5 g of NaCl, 0.2 g of DL-methionine and 15 g of agar in one liter of distilled water and having pH of 7.2) containing 5 µg/ml of chloramphenicol, selecting the intended transformant therefrom. The thus-obtained transformant was referred to as AJ 11082/pSSM30BS.

(Production of L-lysine from sucrose, using reduced sucrase gene-amplified bacteria) AJ 11082 and AJ 11082/pSSM30BS were separately cultured in a medium containing sucrose to produce L-lysine by fermentation. The cells were inoculated in a one-liter jar fermenter containing 300 ml of a lysine-producing medium having sucrose as the carbon source (i.e., containing 100 g of sucrose, 55 g of (NH₄)₂SO₄, 1 g of KH₂PO₄, 1 g of MgSO₄.7H₂O, 50 ml of hydrolysate of soybean protein, 10 mg of FeSO₄.7H₂O, 10 mg of MnSO₄.4H₄0 and 5 mg of nicotinic acid amide in one liter of distilled water) and the cultivation was performed at 31.5°C and at pH of 7.0 (adjusted with ammonia gas) with agitation and aeration. After the sucrose originally added to the medium was wholly consumed by the cells, a mixture comprising 60 g/dl of sucrose and 8 g/dl of ammonium sulfate, that had been sterilized separately, was continuously fed into the fermenter so that the sucrose concentration in the culture might be controlled to fall within the range of from 1 g/dl to 3 g/dl. Thus, the cultivation was continued until 150 ml of the feed solution was consumed. Table 6 shows the cultivation time and the amount of L-lysine accumulated. Due to the amplification of the sucrase gene, the speed at which L-lysine was produced by fermentation of sucrose was noticeably increased. After the cultivation, a crude enzyme was extracted from the cells and its sucrase activity was measured. This is also shown in Table 6.

**Table 6**

| Strain | Cultivation Time (hour) | Amount of L-lysine Accumulated (g/dl) | Sucrase Activity (unit/mg protein) |
|---|---|---|---|
| AJ11082 | 67 | 9.9 | 0.39 |
| AJ11082/pSSM3 0BS | 35 | 9.5 | 4.10 |

### Example 6

### (Introduction of reduced sucrase gene plasmid into wild strain of Brevibacterium lactofermentum)

Reduced sucrase gene plasmid pSSM30BS was introduced into a wild strain of *Brevibacterium lactofermentum* ATCC 13869, using an electric pulse method (see Japanese Patent Application Laid-Open No. 2-207791). The cells were grown on an M-CM2G plate (containing 5 g of glucose, 10 g of polypeptone, 10 g of yeast extract, 5 g of NaCl, 0.2 g of DL-methionine and 15 g of agar in one liter of distilled water and having pH of 7.2) containing 5 µg/ml of chloramphenicol, thereby selecting the intended transformant therefrom. The thus-obtained transformant was referred to as ATCC 13869/pSSM30BS.

### (Production of L-glutamic acid from sucrose, using reduced sucrase gene-amplified bacteria)

ATCC 13869 and ATCC 13869/pSSM30BS were separately cultured in a medium containing sucrose to produce L-glutamic acid by fermentation. The cells were inoculated in a one-liter jar fermenter containing 300 ml of a glutamic acid-producing medium having sucrose as the carbon source (i.e., containing 100 g of sucrose, 15 g of (NH₄)₂SO₄, 2.5 g of KH₂PO₄, 0.4 g of MgSO₄.7H₂O, 50 ml of hydrolysate of soybean protein, 10 mg of FeSO₄.7H₂O, 10 mg of MnSO₄.4H₂O, 350 µg of thiamine.HCl and 3.5 pg of biotin in one liter of distilled water) and the cultivation was performed at 31.5°C and at pH of 6.5 (adjusted with ammonia gas) with agitation and aeration. After the sucrose originally added to the medium was wholly consumed by the cells, a mixture comprising 60 g/dl of sucrose and 8 g/dl of ammonium sulfate, that had been sterilized separately, was continuously fed into the fermenter such that the sucrose concentration in the culture was controlled to fall within the range of from 1 g/dl to 3 g/dl. Thus, the cultivation was continued until 150 ml of the feed solution was consumed. Table 7 shows the cultivation time and the amount of L-glutamic acid accumulated. Due to the amplification of the sucrase gene, the speed at which L-glutamic acid was produced by fermentation of sucrose was noticeably increased.

**Table 7**

| Strain | Cultivation Time (hour) | Amount of L-glutamic Acid Accumulated (g/dl) |
|---|---|---|
| ATCC13869 | 43 | 10.2 |
| ATCC13869/pSSM30BS | 30 | 10.3 |

### [Advantage of the Invention]

According to the present invention, a DNA fragment derived from Coryneform bacteria and containing a gene coding for a protein having sucrase activity and a recombinant DNA containing said DNA fragment and capable of gene amplification in Coryneform bacteria are obtained. By introducing said recombinant DNA into Coryneform bacteria, the sucrose-assimilating activity of the bacteria is enhanced. Using the bacteria having an enhanced sucrose-assimilating activity, one obtains a method for efficiently producing L-amino acids and nucleic acids in a short period of time.

This application is based on Japanese Patent Application No. 046836/1992, filed March 4, 1992, incorporated herein in its entirety by reference.

Obviously, numerous modifications and variations of the present invention are possible in light of the above teachings. It is therefore to be understood that within the scope of the appended claims, the invention may be practiced otherwise than as specifically described herein.

SEQUENCE LISTING

(2) INFORMATION FOR SEQ ID NO:1:

(i) SEQUENCE CHARACTERISTICS:

(A) LENGTH: 24 base pairs

GGGCTGTTAA ATGACCCAAA TGGG

(3) INFORMATION FOR SEQ ID NO:2:

(i) SEQUENCE CHARACTERISTICS:

(A) LENGTH: 24 base pairs

TACATGTGGG AATGCCCTGA TTTG

(4) INFORMATION FOR SEQ ID NO:3:

(i) SEQUENCE CHARACTERISTICS:

(A) LENGTH: 33 base pairs

GATCAAGGTT TTGATTTTTA CGCGCCGCAA ACA

(5) INFORMATION FOR SEQ ID NO:4:

(i) SEQUENCE CHARACTERISTICS:

(A) LENGTH 6911 base pairs

AGTCCGTCGA CGCCACCATT GATGTGGTGG TCACCGAGCT TGCGGAGGCT TTCTACATCT

ACGCTCCCGT CGGCGTGGAG TGGGGTCATT ACGGGTGGGA TCACGCCGGT GAAAGTTGCG

GAACCCATGG TGTTCCTTGT GGGTTGAGGG AACGAGTGCG GGTGAGAAGT TTTTCAAGTG

TCTGCAGTTT TTAAGTTATG CATCATCAGC TTGGAAGGCT GAGGTAATTC AGTAGACCTG

CAACAGCAGG CCTCAAGTCC GAAGATAATT AACCTAGATC CGTAGACATA AGACATCATA

CGTCCTATGC TTGCTGGAAG GAACCAAATA ACCTCAGAAA GATGGCAGAA GTGGTGCATT

ATCAAGAAAA TGCAGGTCAA GCAGTTAAAA AAATTGAGGG AAGAATTGTT CCCCCCCTCG

GGGTGATTGA TGGCTTTCTC CAACTCGAAA ACGGCATCAT CACGGAACTC TCTGGAGAAC

CAGCACCTAA AAACGCAGGA TTCCACCCCG AACTCCCCAC GATTGTTCCC GGTTTTATTG

ATCTTCATAA TCACGGTGGA AACGGTGGCG CGTTTCCTAC GGGAACGCAG GACCAGGCGA

GGAACACCGC GCAGTATCAC CGCGAACATG GCACGACCGT GATGTTGCCA AGCATGGTTT

CGGCGCCGGC TGACGCACTG GCAGCGCAGG TGGAAAACCT TATTCCCTTG TGTGAAGAGG

TCCTGCTGTG CGGCATTCAC CTCGAGGGCC CTTTCATCAA CGCATGCCGT TGTGGTGCTC

AAAACCCGGA TTTCATTTTT CCCGGCAACC CAACAGATCT TGCCCGGGTG ATCCATGCGG

GAAAAGGTTG GATCAAATCG ATCACAGTAG CGCCGGAAAC TGACAATCTT TCTGAGCTTC

TCGATCTCTG CGCAGCGCAC CACATCATTG CTTCCTTCGG GCACACTGAT GCAGATTTTG
ATACCACTAC CAGCGCAATT GCCTTGGCTA AAGAGAAAAA TGTGACGGTC ACGGCTACGC
ATTTGTTCAA TGCGATGCCT CCGCTGCATC ATAGGGCTCC CGGCAGCGTG GGCGCTTTGC
TTGCTGCGGC ACGTGCCGGG GACGCATATG TTGAGTTGAT CGCCGACGGC GTGCATTTGG
CCGATGGAAC GGTCGATCTA GCTCGTTCCA ACAACGCCTT TTTCATCACG GACGCCATGG
AAGCCGCCGG AATGCCAGAC GGTGAGTACA TTTTGGGCGT TTTGAACGTC ACCGTCACCG
ATGGAGTCGC CCGTCTGCGC GATGGCGGCG CCATCGCCGG GGGCACCAGC ACACTAGCGA
GTCAGTTCGT GCACCACGTG CGCAGGGGTA TGACGCTTAT CGACGCGACC CTCCACACCT
CAACCGTCGC CGCTAAAATT CTCGGTCTTG GCGATCACGA AATCGCTAAA TCCAACCCTG
CAAATTTTGT GGTCTTTGAC TCAAACGGCC AGGTGCAAAA GGTCCATTTA GGTCATCAAG
TACTTTAAGT ACGAGTAAAA CTATCCTGAT TTTAAAGGAG TCCCACCATG GAAATCACTA
TCTGCAAAGA CGAGCAAGAA GTCGGCAAAG CAGTTGCAGT CCTAATCGCA CCCTTCGCCA
ACAAGGGTGG AACCTTGGGG CTTGCAACAG GATCCTCACC ACTGAGTACC TACCAAGAGC
TCATTCGCAT GTATGAAGCT GGGGAAGTGT CATTCAAGAA CTGCAAGGCA TTCTTGTTGG
ATGAATACGT GGGACTAACC CGTGACGATG AAAACAGCTA CTTTAAAACC ATTCGCAAAG
AGTTCACTGA CCACATCGAC ATCGTTGATG AAGAGGTCTA CAGCCCAGAT GGTGCAAACC
CTGATCCATA CGAAGCAGCT GCAGAGTATG AGGCAAAGAT CGCTGCAGAA TCCGTTGAAG
TTCAAATCCT TGGCATCGGC GGAAACGGCA CATCGCTTTC ATTGAACCAT CATCTTCTCT
GTCAGGACTG ACAAAGGTCC AGGCGCTGCA CCCTAAAACT GTGGAGGACA ACGCTCGATT
CTTCAACACC ATCGAAGAGG TCCCAACCCA CGCCGTCACC CAGGGTTTGG GCACTTTGTC
CCGCGCGCAA AACATCGTGT TGGTGGCAAC TGGTGAAGGA AAAGCCGACG CCATCCGCGG
AACTGTGGAA GGCCCAGTGA CTGCTTCTTG CCCAGGTTCC ATCCTGTAGA TGCACAACAT
GCCACCATCA TCGTTGGATG AAGCAGCAGT ATCCAAGCTG GAAAACGCTG ATCACTACCG

TCTCATGGAG CAATTAAAGC TGCGCTAGAA ACAAAAAGGA AAGTACTGTG TGGGGCTATG
CACACAGAAC TTTCCAGTTT GCGCCCTGCG TACCATGTGA CTCCTCCGCA GGGCAGGCTC
AATGATCCCA ACGGAATGTA CGTCGATGGA GATACCCTCC ACGTCTACTA CCAGCACGAT
CCAGGTTTCC CCTTCGCACC AAAGCGCACC GGCTGGGCTC ACACCACCAC GCCGTTGACC
GGACCGCAGC GATTGCAGTG GACGCACCTG CCCGACGCTC TTTACCCGGA TGCATCCTAT
GACCTGGATG GATGCTATTC CGGTGGAGCC GTATTTACTG ACGGCACACT TAAACTTTTC
TACACCGGCA ACCTAAAAAT TGACGGAAAG CGCCGCGCCA CCCAAAACCT TGTCGAAGTC
GAGGACCCAA CTGGGCTGAT GGGCGGCATT CATCGCCGTT CGCCTAAAAA TCCGCTTATC
GACGGACCCG CCAGCGGTTT CACACCCCAT TACCGCGATC CCATGATCAG CCCTGATGGT
GATGGTTGGA ACATGGTTCT TGGGGCCCAA CGCGAAAACC TCACCGGTGC AGCGGTTCTA
TACCGCTCGA CAGATCTTGA AAACTGGGAA TTCTCCGGTG AAATCACCTT TGACCTCAGT
GATGCACAAC CTGGTTCTGC TCCTGATCTC GTTCCCGATG GCTACATGTG GGAATGCCCC
AACCTTTTTA CGCTTCGCGA TGAAGAAACT GGCGAAGATC TCGACGTGCT GATTTTCTGT
CCACAAGGAT TGGACCGAAT CCACGATGAG GTTACTCACT ACGCAAGCTC TGACCAGTGC
GGATATGTCG TCGACAAGCT TGAAGGAACG ACCTTCCGCG TCTTGCGAGG ATTCAGCGAG
CTGGATTTCG GCCATGAATT CTACGCACCG CAGGTTGCAG TAAACGGTTC TGATGCCTGG
CTCGTGGGCT GGATGGGGCT GCCCGCGCAG GATGATCACC CAACAGTTGC ACAGGAAGGA
TGGGTGCACT GCCTGACTGT GCCCCGCAAG CTTCATTTGC GCAACCACGC GATCTACCAA
GAGCTCCTTC TCCCAGAGGG GGAGTCGGGG GTAATCAGAT CTGTATTAGG TTCTGAACCT
GTCCGAGTAG ACATCCGAGG CAATATTTCC CTCGAGTGGG ATGGTGTCCG TTTGTCTGTG
GATCGTGATG GTGATCGTCG CGTAGCTGAG GTAAAACCTG GCGAATTAGT GATCGCGGAC
GATAATACAG CCATTGAGAT AACTGCAGGT GATGGACAGG TTTCATTCGC TTTTCCGGGC
CTTCAAAGGT GACACTATTG AGAGATAAGT CATATAAAAG GGTCTTTTGT GGCGAATTGT

ACAAATACTT CGCAAAATCC CTTGATCTAG TTATTGTCAC TGATGACAAC CCTCGTTCAG
AGGTGCCTGC CACGATTCGC GCAGCAGTCA CTGCAGGAGC ACAGCAGGGT GCTTCAGAGT
CCGAACGACC GGTGGAAGTC CTAGAAATTG GTGACCGTGC AGAAGCAATT CGCGTTTTGG
TCGAGTGGGC ACAGCCTGGA GATGGCATTG TAGTAGCTGG AAAAGGCCAT GAAGTTGGAC
AACTAGTTGC TGGTGTCACC CACCATTTTG ATGACCGCGA AGAAGTTCGC GCTGCTTTGA
CAGAAAAGCT CAACAATAAA CTTCCCCTTA CTACGGAAGA AGGATAGGCC ACAGTCATGA
TCACAATGAC CCTTGGGGAA ATCGCTGACA TCGTTGGAGG CAGGCTTACT GGCGGTGCTC
AAGAAGATAC GCTTGTGAGC TCCAGCGTGG AATTTGATTC TCGATCCCTC ACACCGGGTG
GCTTGTTTTT AGCACTTCCG GGTGCTCGTG TAGACGGGCA TGATTTTGCT GCAACTGCAA
TTGAGAAAGG TGCGGTCGCA GTATTGGCAG CCCGTGAGGT TGACGTACCT GCGATCGTCG
TGCCTCCAGT AAAAATCCAG GAATCCAATG CTGACATTTA TGCTCATGAT CCAGATGGGC
ATGGCGCGGC GGTAGTGGAG GCGTTGGTCT CGGTTGGCTC GCCACGTGGT GGATATCTGC
GTGGATGGCC ATCAATTGAA CGTTGTGGCT ATTACTGGTT CTGTGGGAAA GACTTCTATG
AAGGATTTCA TCGCGACGGT TCTTGGCCAA GATGGGCCAA CTGTGGCTCC TCCGGGCTCG
TTTAACAATG AGCTTGGTTT GCCACACACC GCGCTCCGCT GCACAACCGA TACTAAGTAT
TTGGTGGCTG AGATGTCCGC GCGTGGCATT GGACATATTA AGCACCTGAC AGAGATTGCT
CCGCCACGGA TTGCAGCTGT GCTCAACGTC GGCCATGCGC ACCTGGGTGA ATTTGGATCC
CGCGAGAATA TCGCGCAGGC AAAAGGCGAG ATCATTGAAG CGCTGCCCTC GAAGAAAACG
GGTGGGGTAG CAGTCCTTAA CGCTGACGAT CCTTTTGTCG CCCGGATGGC TCCACGCACT
AAGGCGCGCG TGGTGTGGTT TACCACCGAT GCAGGTCAAG CAAAAAAGTC TGATTATTGG
GCAACGAGTA TTTCACTGGA CGCTGTTGCG CGGGCAAGCT TTACGCTGAA CACGAAGGAC
GGGTCTTGGC CGGTCGCCCT GCAGGTTTTT GGTGAGCACC AGGTTGCTAA TGCACTTGCT
GCTGCTGCCA TTGCCATGGA AGCTGGCGTC GCCCCAGAAT TGGTGGTTGC TGGATTGGAA

GCACATTCAG CGGCTTCCGC GCACCGCATG GATGTAAAGA CCCGCGCCGA CGGCGTGACC
ATCATCAACG ATTCTTACAA CGCGAATCCT GATTCTATGC GTGCAGGTAT CGCGGCTCTT
GCGTACACAG CTAGTGGTCG TTCTCTGAAG CAACAAGCTG GGCAGTGCTT GGTCAAATGG
GTGAGCTTGG CGATGACGCC TCGGAAGCCC ATGCCGAACT TGGTGCTGAG CTGCCTAAAT
ACAATGTTCA AGAACTTGTC GCAGTGGGGG AGAACCCTAC CTGTGCAGCA CTTGCAGAGT
CCGCAGCGAG CCTGGGTGTG AGTACTCACG TAGTTTCAGA CGTTGATGCA GCGCTCGAGT
TGCTCGCAGC CCATATTAAG CGGGATGATG TAGTGCTGGT TAAGGCTTCA AATGCTGATC
GCCTGTGGAG GGTCGCAGAA GCACTACATG GCATGGTGCC GGCCTCAAAA ACACAGGTGG
CTCGGTCAAC GACGATTCTC GTCGGAACGT GGAAGGACAG TAGAAAACAA TGCAACAGAT
TATGGTCAGT GGAACGGTTG CGTTCCTCGT CTCAATCTTT CTCACCCCGG TGTTGATCCG
TTATTTCACT AACCGCCAGT TGGGCCAGGA AATCCGTGAA GAAGGCCTGC AGTCTCACTT
GCGTAAGCGT GGCACTCCAA CCATGGGTGG CATTGCGATT ATCGCGGGCA TTGTTGTGGC
CTATGTGTTT ACCAATATCT TGGCCATGAT CCAAGGCGTT GGTGGATTCA CAGTCTCCGG
CTTGCTCGTG TTGGGTCTGA CCTTGGGCCT TGGTGCCACT GGCTTCGCCG ATGACTTCAT
CAAGCTGTAC ATGAACCGAA ACCTTGGTTT GAACAAGACC GCTAAGCTGG TGTCTCAGCT
GGCCATTGCG TTGATCTTTG GTTTTTTGGT ACTGCAGTTT CCCGATGAAA ACGGTCTGAC
CCCAGCATCA ACCCACCTGT CATTCATTCG CGATATCGAC ACCATTGACC TTGGCTTCGG
GGACAGCGTT TTTGGCATCA TCGTGTTCCT CATTTTTATC TACGTTGTGG TCAGCGCGTG
GTCGAATGCC GTGAACATCA CTGATGGTTT GGATGGTTTG GCTGCAGGTA CCACAGCATT
TGTCATGGGT GCTTACACCT TGATCACGTT CTGGCAGTTC CGAAACTCCT GCGATACTGC
AGTGGAAGCG GGTTGCAATA CGGTGCGTGA TCCACTGGAT TTGTCTGTGT TGTGCGCTGC
TGGTCTGGCG CCACCTTGGG CTTTCTGTGG TGGAATGCGG CACCGACAAA GATCTTCATG
GGCGATACTG GTTCTTTGGC ACTGGGCGGT TTGGTTGCAG GTATTTCTGT GGTTAGCCGC

ACCGAGCTGC TCATGGTTAT CATCGGCGCG CTGTTTGTCA TTGAGGTCGC TTCTGTTGCG
ATCCAGATCG GCGTGTTTAA GACCCGCGGT AAGCGTGTGT TCAAAATGGC TCCGATCCAC
CACCACTTCG AGGCCCTTGG GTGGACTGAA ACTACCGTGA CCATCCGTTT CTGGCTGATC
ACGATCATGA CTGTGTTGGC GGGTGTCGGT GTGTTTAACA GCGACTGGCT CCACTTAGCG
GAGGTATAAA TAATTATGGT TTCTCTGTCC CATTTACCTC AGGCGCTGCA GGGCCGTATT
CTTGTGGCCG GCGCTGGTGT TTCCGGCCTG TCCATAGCAA AGATGCTCAG TGAGTTGCAT
TGCGATGTTG TGGTCACCGA CGAGAACGAA ACTGCACGTC ACATGCTCAT TGAAGTAGTA
GACGTTGCAG ATATCAGCAC CGCCCAGGCT CAGGAACAGC TGGATTCTTT CTCCATTGTG
GTCACCTCCC C

## Claims

1. A DNA fragment derived from Coryneform bacteria and containing a gene coding for a protein having sucrase activity.

2. A DNA fragment according to claim 1, wherein the gene comprises the sequence from bases 2,338 to 3,609 of base sequence ID NO:4.

3. A DNA fragment containing a gene coding for a protein having amino acid substitutions and/or insertions and/or deletions with respect to the protein encoded by the sequence as defined in claim 2 and having at least 75 % of the sucrase activity of the protein encoded by the sequence as defined in claim 2.

4. A vector, in which the DNA fragment according to any of claims 1 to 3 is operably linked to regulatory DNA effecting expression of said gene.

5. A microorganism, in which the DNA fragment according to any of claims 1 to 3 is operably linked to regulatory DNA effecting expression of said gene.

6. A microorganism according to claim 5, which belongs to the genus Coryneform bacteria.

7. A microorganism according to claim 5 or 6, which is capable of producing an L-amino acid or a nucleic acid.

8. A method for producing an L-amino acid or a nucleic acid, wherein the microorganism according to any of claims 5 to 7 is cultured in a liquid medium containing sucrose to produce and accumulate an L-amino acid or a nucleic acid in the culture.

9. The method according to claim 8, in which the L-amino acid is L-lysine.

10. The method according to claim 8, in which the L-amino acid is L-glutamic acid.


SEQUENCE LISTING

(1) GENERAL INFORMATION:

(i) APPLICANT:
(A) NAME: Ajinomoto Co., Inc.
(B) STREET: No. 15-1, Kyobashi 1-chome, Chuo-ku
(C) CITY: Tokyo
(E) COUNTRY: Japan
(F) POSTAL CODE (ZIP): 104
(G) TELEPHONE: (03)5250-8178
(H) TELEFAX: (03)5250-8347
(I) TELEX: J22690

(ii) TITLE OF INVENTION: Sucrase gene derived from Coryneform bacteria
(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96101228.3

(2) INFORMATION FOR SEQ ID NO:1:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: Other nucleic acid

(xi) SEQUENCE DESCRIPTION: SEQ ID NO:1:

GGGCTGTTAA ATGACCCAAA TGGG 24

(2) INFORMATION FOR SEQ ID NO:2:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: Other nucleic acid

(xi) SEQUENCE DESCRIPTION: SEQ ID NO:2:

TACATGTGGG AATGCCCTGA TTTG 24

(2) INFORMATION FOR SEQ ID NO:3:

(i) SEQUENCE CHARACTERISTICS:
(A) LENGTH: 33 base pairs
(B) TYPE: nucleic acid
(C) STRANDEDNESS: single
(D) TOPOLOGY: unknown

(ii) MOLECULE TYPE: Other nucleic acid

(xi) SEQUENCE DESCRIPTION: SEQ ID NO:3:

GATCAAGGTT TTGATTTTTA CGCGCCGCAA ACA 33

(2) INFORMATION FOR SEQ ID NO:4:

(i) SEQUENCE CHARACTERISTICS:
(A) LENGTH: 6911 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:4:

AGTCCGTCGA CGCCACCATT GATGTGGTGG TCACCGAGCT TGCGGAGGCT TTCTACATCT 60

ACGCTCCCGT CGGCGTGGAG TGGGGTCATT ACGGGTGGGA TCACGCCGGT GAAAGTTGCG 120

GAACCCATGG TGTTCCTTGT GGGTTGAGGG AACGAGTGCG GGTGAGAAGT TTTTCAAGTG 180

TCTGCAGTTT TTAAGTTATG CATCATCAGC TTGGAAGGCT GAGGTAATTC AGTAGACCTG 240

CAACAGCAGG CCTCAAGTCC GAAGATAATT AACCTAGATC CGTAGACATA AGACATCATA 300

CGTCCTATGC TTGCTGGAAG GAACCAAATA ACCTCAGAAA GATGGCAGAA GTGGTGCATT 360

ATCAAGAAAA TGCAGGTCAA GCAGTTAAAA AAATTGAGGG AAGAATTGTT CCCCCCCTCG 420

GGGTGATTGA TGGCTTTCTC CAACTCGAAA ACGGCATCAT CACGGAACTC TCTGGAGAAC 480

CAGCACCTAA AAACGCAGGA TTCCACCCCG AACTCCCCAC GATTGTTCCC GGTTTTATTG 540

ATCTTCATAA TCACGGTGGA AACGGTGGCG CGTTTCCTAC GGGAACGCAG GACCAGGCGA 600

GGAACACCGC GCAGTATCAC CGCGAACATG GCACGACCGT GATGTTGCCA AGCATGGTTT 660

CGGCGCCGGC TGACGCACTG GCAGCGCAGG TGGAAAACCT TATTCCCTTG TGTGAAGAGG 720

TCCTGCTGTG CGGCATTCAC CTCGAGGGCC CTTTCATCAA CGCATGCCGT TGTGGTGCTC 780

AAAACCCGGA TTTCATTTTT CCCGGCAACC CAACAGATCT TGCCCGGGTG ATCCATGCGG 840

GAAAAGGTTG GATCAAATCG ATCACAGTAG CGCCGGAAAC TGACAATCTT TCTGAGCTTC 900

TCGATCTCTG CGCAGCGCAC CACATCATTG CTTCCTTCGG GCACACTGAT GCAGATTTTG 960

ATACCACTAC CAGCGCAATT GCCTTGGCTA AAGAGAAAAA TGTGACGGTC ACGGCTACGC 1020

ATTTGTTCAA TGCGATGCCT CCGCTGCATC ATAGGGCTCC CGGCAGCGTG GGCGCTTTGC 1080

TTGCTGCGGC ACGTGCCGGG GACGCATATG TTGAGTTGAT CGCCGACGGC GTGCATTTGG 1140

CCGATGGAAC GGTCGATCTA GCTCGTTCCA ACAACGCCTT TTTCATCACG GACGCCATGG 1200

AAGCCGCCGG AATGCCAGAC GGTGAGTACA TTTTGGGCGT TTTGAACGTC ACCGTCACCG 1260

ATGGAGTCGC CCGTCTGCGC GATGGCGGCG CCATCGCCGG GGGCACCAGC ACACTAGCGA 1320

GTCAGTTCGT GCACCACGTG CGCAGGGGTA TGACGCTTAT CGACGCGACC CTCCACACCT 1380

CAACCGTCGC CGCTAAAATT CTCGGTCTTG GCGATCACGA AATCGCTAAA TCCAACCCTG 1440

CAAATTTTGT GGTCTTTGAC TCAAACGGCC AGGTGCAAAA GGTCCATTTA GGTCATCAAG 1500

TACTTTAAGT ACGAGTAAAA CTATCCTGAT TTTAAAGGAG TCCCACCATG GAAATCACTA 1560

TCTGCAAAGA CGAGCAAGAA GTCGGCAAAG CAGTTGCAGT CCTAATCGCA CCCTTCGCCA 1620

ACAAGGGTGG AACCTTGGGG CTTGCAACAG GATCCTCACC ACTGAGTACC TACCAAGAGC 1680

TCATTCGCAT GTATGAAGCT GGGGAAGTGT CATTCAAGAA CTGCAAGGCA TTCTTGTTGG 1740

ATGAATACGT GGGACTAACC CGTGACGATG AAAACAGCTA CTTTAAAACC ATTCGCAAAG 1800

AGTTCACTGA CCACATCGAC ATCGTTGATG AAGAGGTCTA CAGCCCAGAT GGTGCAAACC 1860

CTGATCCATA CGAAGCAGCT GCAGAGTATG AGGCAAAGAT CGCTGCAGAA TCCGTTGAAG 1920

TTCAAATCCT TGGCATCGGC GGAAACGGCA CATCGCTTTC ATTGAACCAT CATCTTCTCT 1980

GTCAGGACTG ACAAAGGTCC AGGCGCTGCA CCCTAAAACT GTGGAGGACA ACGCTCGATT 2040

CTTCAACACC ATCGAAGAGG TCCCAACCCA CGCCGTCACC CAGGGTTTGG GCACTTTGTC 2100

CCGCGCGCAA AACATCGTGT TGGTGGCAAC TGGTGAAGGA AAAGCCGACG CCATCCGCGG 2160

AACTGTGGAA GGCCCAGTGA CTGCTTCTTG CCCAGGTTCC ATCCTGTAGA TGCACAACAT 2220

GCCACCATCA TCGTTGGATG AAGCAGCAGT ATCCAAGCTG GAAAACGCTG ATCACTACCG 2280

TCTCATGGAG CAATTAAAGC TGCGCTAGAA ACAAAAAGGA AAGTACTGTG TGGGGCTATG 2340

CACACAGAAC TTTCCAGTTT GCGCCCTGCG TACCATGTGA CTCCTCCGCA GGGCAGGCTC 2400

AATGATCCCA ACGGAATGTA CGTCGATGGA GATACCCTCC ACGTCTACTA CCAGCACGAT 2460

CCAGGTTTCC CCTTCGCACC AAAGCGCACC GGCTGGGCTC ACACCACCAC GCCGTTGACC 2520

GGACCGCAGC GATTGCAGTG GACGCACCTG CCCGACGCTC TTTACCCGGA TGCATCCTAT 2580

GACCTGGATG GATGCTATTC CGGTGGAGCC GTATTTACTG ACGGCACACT TAAACTTTTC 2640

TACACCGGCA ACCTAAAAAT TGACGGAAAG CGCCGCGCCA CCCAAAACCT TGTCGAAGTC 2700

GAGGACCCAA CTGGGCTGAT GGGCGGCATT CATCGCCGTT CGCCTAAAAA TCCGCTTATC 2760

GACGGACCCG CCAGCGGTTT CACACCCCAT TACCGCGATC CCATGATCAG CCCTGATGGT 2820

GATGGTTGGA ACATGGTTCT TGGGGCCCAA CGCGAAAACC TCACCGGTGC AGCGGTTCTA 2880
TACCGCTCGA CAGATCTTGA AAACTGGGAA TTCTCCGGTG AAATCACCTT TGACCTCAGT 2940
GATGCACAAC CTGGTTCTGC TCCTGATCTC GTTCCCGATG GCTACATGTG GGAATGCCCC 3000
AACCTTTTTA CGCTTCGCGA TGAAGAAACT GGCGAAGATC TCGACGTGCT GATTTTCTGT 3060
CCACAAGGAT TGGACCGAAT CCACGATGAG GTTACTCACT ACGCAAGCTC TGACCAGTGC 3120
GGATATGTCG TCGACAAGCT TGAAGGAACG ACCTTCCGCG TCTTGCGAGG ATTCAGCGAG 3180
CTGGATTTCG GCCATGAATT CTACGCACCG CAGGTTGCAG TAAACGGTTC TGATGCCTGG 3240
CTCGTGGGCT GGATGGGGCT GCCCGCGCAG GATGATCACC CAACAGTTGC ACAGGAAGGA 3300
TGGGTGCACT GCCTGACTGT GCCCCGCAAG CTTCATTTGC GCAACCACGC GATCTACCAA 3360
GAGCTCCTTC TCCCAGAGGG GGAGTCGGGG GTAATCAGAT CTGTATTAGG TTCTGAACCT 3420
GTCCGAGTAG ACATCCGAGG CAATATTTCC CTCGAGTGGG ATGGTGTCCG TTTGTCTGTG 3480
GATCGTGATG GTGATCGTCG CGTAGCTGAG GTAAAACCTG GCGAATTAGT GATCGCGGAC 3540
GATAATACAG CCATTGAGAT AACTGCAGGT GATGGACAGG TTTCATTCGC TTTTCCGGGC 3600
CTTCAAAGGT GACACTATTG AGAGATAAGT CATATAAAAG GGTCTTTTGT GGCGAATTGT 3660
ACAAATACTT CGCAAAATCC CTTGATCTAG TTATTGTCAC TGATGACAAC CCTCGTTCAG 3720
AGGTGCCTGC CACGATTCGC GCAGCAGTCA CTGCAGGAGC ACAGCAGGGT GCTTCAGAGT 3780
CCGAACGACC GGTGGAAGTC CTAGAAATTG GTGACCGTGC AGAAGCAATT CGCGTTTTGG 3840
TCGAGTGGGC ACAGCCTGGA GATGGCATTG TAGTAGCTGG AAAAGGCCAT GAAGTTGGAC 3900
AACTAGTTGC TGGTGTCACC CACCATTTTG ATGACCGCGA AGAAGTTCGC GCTGCTTTGA 3960
CAGAAAAGCT CAACAATAAA CTTCCCCTTA CTACGGAAGA AGGATAGGCC ACAGTCATGA 4020
TCACAATGAC CCTTGGGGAA ATCGCTGACA TCGTTGGAGG CAGGCTTACT GGCGGTGCTC 4080
AAGAAGATAC GCTTGTGAGC TCCAGCGTGG AATTTGATTC TCGATCCCTC ACACCGGGTG 4140
GCTTGTTTTT AGCACTTCCG GGTGCTCGTG TAGACGGGCA TGATTTTGCT GCAACTGCAA 4200
TTGAGAAAGG TGCGGTCGCA GTATTGGCAG CCCGTGAGGT TGACGTACCT GCGATCGTCG 4260
TGCCTCCAGT AAAAATCCAG GAATCCAATG CTGACATTTA TGCTCATGAT CCAGATGGGC 4320
ATGGCGCGGC GGTAGTGGAG GCGTTGGTCT CGGTTGGCTC GCCACGTGGT GGATATCTGC 4380
GTGGATGGCC ATCAATTGAA CGTTGTGGCT ATTACTGGTT CTGTGGGAAA GACTTCTATG 4440
AAGGATTTCA TCGCGACGGT TCTTGGCCAA GATGGGCCAA CTGTGGCTCC TCCGGGCTCG 4500
TTTAACAATG AGCTTGGTTT GCCACACACC GCGCTCCGCT GCACAACCGA TACTAAGTAT 4560
TTGGTGGCTG AGATGTCCGC GCGTGGCATT GGACATATTA AGCACCTGAC AGAGATTGCT 4620

CCGCCACGGA TTGCAGCTGT GCTCAACGTC GGCCATGCGC ACCTGGGTGA ATTTGGATCC 4680
CGCGAGAATA TCGCGCAGGC AAAAGGCGAG ATCATTGAAG CGCTGCCCTC GAAGAAAACG 4740
GGTGGGGTAG CAGTCCTTAA CGCTGACGAT CCTTTTGTCG CCCGGATGGC TCCACGCACT 4800
AAGGCGCGCG TGGTGTGGTT TACCACCGAT GCAGGTCAAG CAAAAAAGTC TGATTATTGG 4860
GCAACGAGTA TTTCACTGGA CGCTGTTGCG CGGGCAAGCT TTACGCTGAA CACGAAGGAC 4920
GGGTCTTGGC CGGTCGCCCT GCAGGTTTTT GGTGAGCACC AGGTTGCTAA TGCACTTGCT 4980
GCTGCTGCCA TTGCCATGGA AGCTGGCGTC GCCCCAGAAT TGGTGGTTGC TGGATTGGAA 5040
GCACATTCAG CGGCTTCCGC GCACCGCATG GATGTAAAGA CCCGCGCCGA CGGCGTGACC 5100
ATCATCAACG ATTCTTACAA CGCGAATCCT GATTCTATGC GTGCAGGTAT CGCGGCTCTT 5160
GCGTACACAG CTAGTGGTCG TTCTCTGAAG CAACAAGCTG GGCAGTGCTT GGTCAAATGG 5220
GTGAGCTTGG CGATGACGCC TCGGAAGCCC ATGCCGAACT TGGTGCTGAG CTGCCTAAAT 5280
ACAATGTTCA AGAACTTGTC GCAGTGGGGG AGAACCCTAC CTGTGCAGCA CTTGCAGAGT 5340
CCGCAGCGAG CCTGGGTGTG AGTACTCACG TAGTTTCAGA CGTTGATGCA GCGCTCGAGT 5400
TGCTCGCAGC CCATATTAAG CGGGATGATG TAGTGCTGGT TAAGGCTTCA AATGCTGATC 5460
GCCTGTGGAG GGTCGCAGAA GCACTACATG GCATGGTGCC GGCCTCAAAA ACACAGGTGG 5520
CTCGGTCAAC GACGATTCTC GTCGGAACGT GGAAGGACAG TAGAAAACAA TGCAACAGAT 5580
TATGGTCAGT GGAACGGTTG CGTTCCTCGT CTCAATCTTT CTCACCCCGG TGTTGATCCG 5640
TTATTTCACT AACCGCCAGT TGGGCCAGGA AATCCGTGAA GAAGGCCTGC AGTCTCACTT 5700
GCGTAAGCGT GGCACTCCAA CCATGGGTGG CATTGCGATT ATCGCGGGCA TTGTTGTGGC 5760
CTATGTGTTT ACCAATATCT TGGCCATGAT CCAAGGCGTT GGTGGATTCA CAGTCTCCGG 5820
CTTGCTCGTG TTGGGTCTGA CCTTGGGCCT TGGTGCCACT GGCTTCGCCG ATGACTTCAT 5880
CAAGCTGTAC ATGAACCGAA ACCTTGGTTT GAACAAGACC GCTAAGCTGG TGTCTCAGCT 5940
GGCCATTGCG TTGATCTTTG GTTTTTTGGT ACTGCAGTTT CCCGATGAAA ACGGTCTGAC 6000
CCCAGCATCA ACCCACCTGT CATTCATTCG CGATATCGAC ACCATTGACC TTGGCTTCGG 6060
GGACAGCGTT TTTGGCATCA TCGTGTTCCT CATTTTTATC TACGTTGTGG TCAGCGCGTG 6120
GTCGAATGCC GTGAACATCA CTGATGGTTT GGATGGTTTG GCTGCAGGTA CCACAGCATT 6180
TGTCATGGGT GCTTACACCT TGATCACGTT CTGGCAGTTC CGAAACTCCT GCGATACTGC 6240
AGTGGAAGCG GGTTGCAATA CGGTGCGTGA TCCACTGGAT TTGTCTGTGT TGTGCGCTGC 6300
TGGTCTGGCG CCACCTTGGG CTTTCTGTGG TGGAATGCGG CACCGACAAA GATCTTCATG 6360
GGCGATACTG GTTCTTTGGC ACTGGGCGGT TTGGTTGCAG GTATTTCTGT GGTTAGCCGC 6420

ACCGAGCTGC TCATGGTTAT CATCGGCGCG CTGTTTGTCA TTGAGGTCGC TTCTGTTGCG 6480

ATCCAGATCG GCGTGTTTAA GACCCGCGGT AAGCGTGTGT TCAAAATGGC TCCGATCCAC 6540

CACCACTTCG AGGCCCTTGG GTGGACTGAA ACTACCGTGA CCATCCGTTT CTGGCTGATC 6600

ACGATCATGA CTGTGTTGGC GGGTGTCGGT GTGTTTAACA GCGACTGGCT CCACTTAGCG 6660

GAGGTATAAA TAATTATGGT TTCTCTGTCC CATTTACCTC AGGCGCTGCA GGGCCGTATT 6720

CTTGTGGCCG GCGCTGGTGT TTCCGGCCTG TCCATAGCAA AGATGCTCAG TGAGTTGCAT 6780

TGCGATGTTG TGGTCACCGA CGAGAACGAA ACTGCACGTC ACATGCTCAT TGAAGTAGTA 6840

GACGTTGCAG ATATCAGCAC CGCCCAGGCT CAGGAACAGC TGGATTCTTT CTCCATTGTG 6900

GTCACCTCCC C 6911